# EUROPEAN PATENT APPLICATION

(11) **EP 4 686 945 A1**
(43) Date of publication of application: **04.02.2026**
(21) Application number: 24192111.3
(22) Date of filing: 31.07.2024
(51) Int. Cl.: G01N 33/92, C12Q 1/44

(54) **LIVE CELL REAL-TIME LIPOLYSIS DETERMINATION**

(71) Applicant: Eberhard Karls Universität Tübingen Medizinische Fakultät, 72074 Tübingen (DE)
(72) Inventor: Sancar, Gencer, 72076 Tübingen (DE); Birkenfeld, Andreas, 72076 Tübingen (DE); Seigner, Judith, 72076 Tübingen (DE); Krier, Johannes, 72076 Tübingen (DE)
(74) Representative: Witte, Weller & Partner Patentanwälte mbB

(57) **Abstract**

The present invention relates to a method for determining the lipolytic status in a living biological cell.

## Description

The present invention relates to a method for determining the lipolytic status in a living biological cell.

### FIELD OF THE INVENTION

The present invention relates to the field of lipolysis determination, more specific to the determining of the lipolytic status in living biological cells in real-time.

### BACKGROUND

A multicellular organism needs to ensure sufficient availability of energy for its biological cells. An important energy-containing substance is fat, especially triacylglyceride (TAG), which serves as long-term energy storage for the organism as lipid droplets. By metabolic degradation of fats via so-called lipolysis, biological cells can access the stored energy to keep all cellular processes running.

Determination of lipolysis is not only of interest for a multitude of scientific questions, but also for pharmaceutical research. For instance, lipolysis and resistance to insulin are linked since insulin, secreted from pancreas when blood-sugar levels are high, i.e., energy is highly available in blood, induces lipogenesis, which equals storage of energy as low-accessible TAG. Thus, in pharmaceutical research, lipolysis is a measure of insulin resistance or sensitivity.

Methods of prior art for determination of lipolysis of biological cells rely on biochemical assays as for example determining the activity of lipolysis-related enzymes, for example the TG hydrolase activity or measurement of free-fatty-acids (FFAs) released into the medium. However, such assays usually represent end-point determinations, i.e., require destruction of the respective biological cell or at least an ending of the experiment or the respective biological cell culture. Thus, such assay is to be conducted repeatedly on individual aliquots for covering a time period, which is laborious, time consuming, material consuming, and expensive. Moreover, conditions do vary among repeated assays, leading to losses in accuracy.

Diaz et al., Hydrophobic characterization of intracellular lipids in situ by Nile Red red/yellow emission ratio, Micron. 2008 Oct;39(7):819-24, disclose a method for discrimination of specific lipids by comparing differences in the ratio of red to yellow fluorescence emission by Nile Red solvated by those lipids. The method is also applicable to fixed biological cells for analysis of the composition of lipid droplets. The fluorescence spectrum of Nile Red is dependent on the polarity of the lipids solvating it. This solvatochromic effect is used by determining two spectra from Nile Red at different wave lengths. However, the method is limited to fixed biological cells since in living biological cells, the spatial distribution of the lipids is not sufficiently stable to allow reliable spatial discrimination of the lipids. The known method, therefore, does not allow, for example, an examination of lipolysis over time.

Maulucci et al., Real time quantitative analysis of lipid storage and lipolysis pathways by confocal spectral imaging of intracellular micropolarity, Biochim Biophys Acta Mol Biological Cell Biol Lipids, 2018 Jul;1863(7):783-793, disclose a method for discrimination of different lipids in living biological cells to image in real-time lipid droplet formation and lipid remodelling. By application of an algorithm, a single fluorescence spectrum from Nile Red stained living biological cells of each pixel is analysed to identify properties of the composition of lipids solvating the Nile Red at the respective spatial position. However, the method requires a confocal microscope for high-resolution imaging and conduction of a complex algorithm. Therefore, the known method is costly, time-consuming and complex.

Against this background, it is an object of the present invention to overcome the disadvantages and drawbacks of the prior art. Especially, it is an object of the present invention to provide for an easy-to-apply and inexpensive method for determining lipolysis over a prolonged period of time.

This object is fully solved by the present invention.

### SUMMARY OF THE INVENTION

In an aspect of the invention, the above-identified disadvantages are overcome by providing a method for determining the lipolytic status in a living biological cell, the method comprising
(a) contacting a biological cell and Nile Red,
(b) exciting said Nile Red for fluorescence at a first excitation wave length,
(c) determining a fluorescence intensity A emitted by said Nile Red at a first emission wave length,
(d) exciting said Nile Red for fluorescence at a second excitation wave length,
(e) determining a fluorescence intensity B emitted by said Nile Red at a second emission wave length, and
(f) calculating a ratio from the fluorescence intensity A and the fluorescence intensity B,
wherein the ratio calculated in step (f) is indicative of the lipolytic status of the living biological cell.

Contacting a biological cell with Nile Red, as clear to the skilled person, is to be understood as staining a viable or living biological cell with Nile Red. The biological cell may have been cultured before and usually the staining is performed within a suitable buffer, i.e., nutrition medium or culture medium.

By "lipolytic status" as used herein is meant the current status of lipolysis in the cell, i.e. the current propensity of the cell to synthesise non-polar lipids, especially TAG or to degrade the latter under synthesis of polar lipids, especially FFA. However, in one embodiment, the lipolytic status includes lipolysis as a dynamic process in the cell over time.

According to the invention, a "biological cell" comprises any cell which can have a lipolytic status, in particular, but not exclusively, an adipocyte, a hepatocyte, and a muscle cell.

"Calculating a ratio from the fluorescence intensity A and the fluorescence intensity B" is to be understood such that the quotient fluorescence intensity A/fluorescence intensity B or the quotient fluorescence intensity B/fluorescence intensity A is determined.

Nile Red is used in prior art for staining lipids or biological cells and determining the polarity of lipids solvating the Nile Red or localizing different types of lipids associated with certain cellular organelles by use of the solvatochromic effect of Nile Red. In the presence of non-polar lipids, as for example TAG, which are for example present in lipid droplets, Nile Red emits green fluorescence in response to excitation. In the presence of polar lipids, as for example FFAs, Nile Red emits red fluorescence in response to excitation.

The inventors surprisingly found that without the need for complicated high-resolution imaging, the solvatochromic effect of Nile Red can be used to determine the lipolytic status in living biological cells. The method is advantageously applicable with standard laboratory equipment and is conducted easily and fast.

Advantageously, the method allows intracellular determination of the lipolytic status or lipolysis in living biological cells in real-time over a period of at least 8 hours without the need for taking and analysing aliquots or repeated conducting of biochemical assays.

A further advantage is that the sample to be analysed is easily to be manipulated, for example by administering substances to the culture medium, while at the same time determining of the lipolytic status and observing effects of the substances on lipolysis in real-time.

In another embodiment, the method comprises the following further step after step f):
(g) repeating steps (b) to (f) for an at least second round, wherein a period of time t has elapsed between the first round and the at least second round.

This advantageously allows to determine the lipolysis in the living biological cell over a period of time, which allows live cell lipolysis determination.

In another embodiment, the method comprises the following further step after step (g):
(h) comparing the ratio calculated in step (f) in the first round with the ratio calculated in step (f) in the at least second round to determine the lipolytic status of the living biological cell over the time t.

This advantageously allows determination of lipolytic activity in the at least second round relative to the first round, i.e., within a single sample in response to a treatment or manipulation and without a need for a standard.

In another embodiment, the method comprises calculating an integral of the ratio calculated in step (f) of each round over the time t, wherein the integral is indicative of the lipolytic status of the living biological cell over the time t.

This advantageously allows a generalizing determination of lipolysis in the biological cell over a certain period of time and thus a determination of lipolysis relative to other experiments conducting lipolysis determination according to the method.

In another embodiment, the method comprises the following further step before step (a):
(a') administering to the biological cell a substance inducing lipolysis, the substance being preferably selected from the group consisting of: β-adrenergic agonists, alpha-2-antagonists, PDE inhibitors, forskolin, and hormones, more preferably selected from the group consisting of: isoproterenol, PDE inhibitors, forskolin, catecholamines, glucagon, atrial natriuretic peptide (ANP), and brain natriuretic peptide (BNP).

This advantageously allows to yield an observable level of lipolysis since it ensures a basic level of ongoing lipolysis to be conducted by the biological cell.

In another embodiment, the biological cell is an adipocyte.

This is advantageous since for adipocytes conducting lipolysis (or lipogenesis) is of prominent importance for their function or is a main function of the respective cell. Especially, adipocytes naturally store TAG and/or conduct lipolysis.

In another embodiment, the first excitation wave length is from 565 nm to 605 nm, preferably from 575 nm to 595 nm, most preferably 585 nm.

This is advantageously the excitation-maximum of Nile Red that is bound to polar lipids, as FFA.

In another embodiment, the first emission wave length is from 610 nm to 655 nm, preferably from 625 nm to 645 nm, most preferably 635 nm.

This is advantageously the emission-maximum of Nile Red that is bound to polar lipids, as FFA.

In another embodiment, the second excitation wave length is from 440 nm to 490 nm, preferably from 450 nm to 470 nm, most preferably 460 nm.

This is advantageously the excitation-maximum of Nile Red that is bound to non-polar lipids, as TAG, and is advantageously sufficiently far away from the first excitation wave length as to avoid interferences.

In another embodiment, the second emission wave length is from 500 nm to 550 nm, preferably from 515 nm to 535 nm, most preferably 524 nm.

This is advantageously the emission-maximum of Nile Red that is bound to non-polar lipids, as TAG.

In another embodiment, the steps (b) to (f) are repeated for approximately 100 rounds, preferably for approximately 10 rounds.

This allows determination of the dynamics of the lipolysis in the biological cell, for example in response to administration of manipulating substances during an experiment.

In another embodiment, each ratio calculated in step (f) of any round is normalized to a ratio calculated in step (f) of a reference round.

This advantageously makes all ratios calculated in step (f) of any round comparable to each other in relation to the reference round.

In another embodiment, the reference round is the first round.

This is advantageous because this way, the lipolytic status in the living biological cell can be determined relative to the start of an experiment. Changes and/or dynamics over time and/or in response to manipulating substances or other manipulations on the biological cell can, thus, be determined.

In another embodiment, at least the step (f) and/or, if provided, the step (h) is computer-implemented.

This is advantageous for acceleration of the calculation.

In another embodiment, at least one of the steps (b) to (e) is conducted by means of a fluorescence microscope or plate reader, preferably the steps (b) to (e).

This advantageously allows to conduct the method with standard laboratory equipment, easily, and fast.

In another embodiment, the method comprises the step
(s) administering to the biological cell an agent to be tested for its ability to modulate the lipolytic status.

This advantageously allows to identify a new agent showing lipolysis modulating effects, especially when the method is conducted in a high-throughput setting.

In another embodiment, the method comprises the step
(t) administering to the biological cell a modulating agent modulating the lipolytic status.

This advantageously allows to determine the lipolysis in response to administration of a modulating agent.

In this embodiment, the modulating agent is preferably an anti-lipolytic hormone, more preferably selected from the group consisting of: insulin, fibroblast growth factor 1 (FGF-1), and insulin like growth factor 1 (IGF1).

In this embodiment, the method preferably comprises the step u) administering to the biological cell a target to be tested for its ability to antagonize, inhibit, agonize, or induce the modulation of the lipolytic status caused by the modulating agent.

This is advantageous for testing or screening one or more targets for its ability to antagonize, inhibit, agonize, or induce the modulation of the lipolytic status and/or to compare such effects of multiple targets.

In this embodiment, the target is preferably selected from the group consisting of: a potential insulin-sensitizer, a potential insulin-desensitizer, a potential lipase inhibitor, a potential lipolysis activator, and a potential lipolysis inhibitor.

This advantageously allows to test or screen for new medicaments, especially for diabetes medicaments.

It is understood that the features mentioned above and those yet to be explained below can be used not only in the particular combination given, but also in other combinations or in isolation, without departing from the scope of the present invention.

The invention is now described and explained in further detail by referring to the following non-limiting examples and drawings.

### BRIEF DESCRIPTION OF THE FIGURES

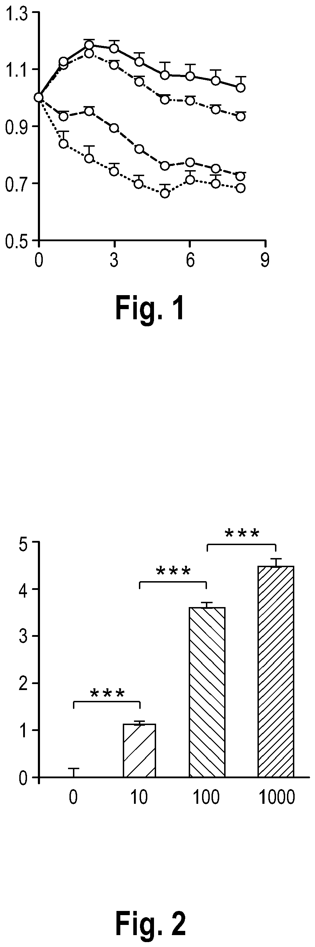

Fig. 1: Diagram of the lipolytic status in 3T3L1 adipocytes after administration of 0 nM (dotted line), 10 nM (dashed line), 100 nM (dotted-dashed line), or 1000 nM (full line) isoproterenol and determination of the lipolysis according to the invention over a period of 8 h each hour, i.e., in 9 rounds, normalized to the ratio calculated in the first round, wherein the y-axis shows the ratio from fluorescence intensity A and fluorescence intensity B, and the x-axis shows the time in hours.
Fig. 2: Diagram of the lipolytic status in 3T3L1 adipocytes after administration of 0 nM, 10 nM, 100 nM, or 1000 nM isoproterenol and determination of the lipolysis according to the invention over a period of 8 h each hour, i.e., in 9 rounds, wherein the y-axis shows the integral of the ratio calculated in step (f) each round over the time normalized to the sample with administration of 0 nM isoproterenol, and the x-axis shows the concentration of isoproterenol in nM.

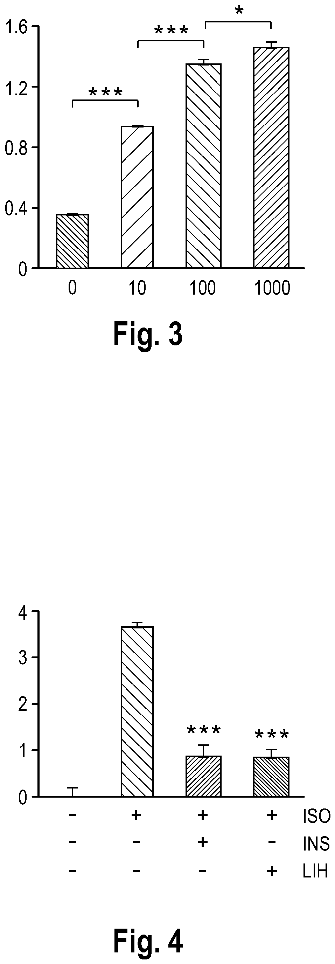

Fig. 3: Diagram of concentration of FFA in the supernatants from samples from Fig. 1 determined by a colorimetric kit after 5 h, wherein the y-axis shows the concentration of FFA and the x-axis shows the concentration of isoproterenol.
Fig. 4: Diagram of the lipolytic status in 3T3L1 adipocytes after administration of isoproterenol (ISO), insulin (INS), and/or a lipase inhibitor (LIH) and determination of the lipolysis according to the invention over a period of 8 h each hour, i.e., in 9 rounds, wherein the y-axis shows the integral of the ratio calculated in step (f) of each round over the time normalized to the sample without administration of isoproterenol, and the x-axis shows the treatment conditions.

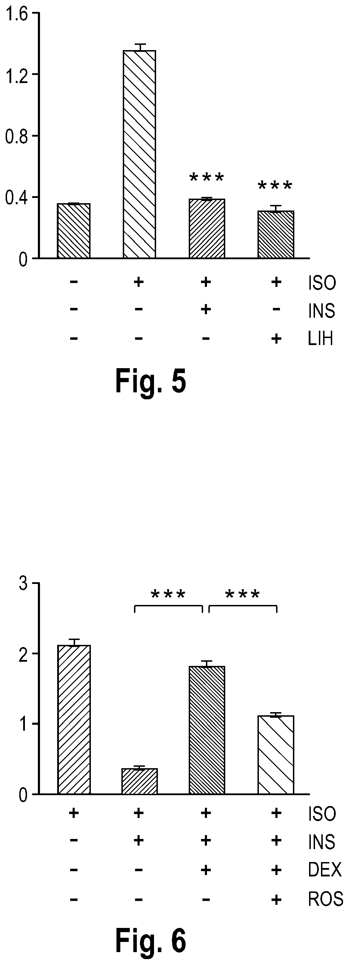

Fig. 5: Diagram of concentration of FFA in the supernatants from samples from Fig. 4 determined by a colorimetric kit after 5 h, wherein the y-axis shows the concentration of FFA and the x-axis shows the treatment conditions.
Fig. 6: Diagram of the lipolytic status in 3T3L1 adipocytes after administration of isoproterenol (ISO), insulin (INS), dexamethasone (DEX) and/or rosiglitazone (ROS) and determination of the lipolysis according to the invention over a period of 8 h each hour, i.e., in 9 rounds, wherein the y-axis shows the integral of the ratio calculated in step (f) of each round over the time normalized to the sample without administration of isoproterenol, and the x-axis shows the treatment conditions.

### EXAMPLES

### 1. Materials and methods

### 1.1 Adipocyte differentiation

3T3L1 pre-adipocytes can be grown to full confluence in DMEM supplemented with 10% FBS, 10 mM Hepes, and antibiotic-antimycotic (growth media) in 48-well biological cell culture plates. Differentiation can be induced by replacing the media by 1 µM dexamethasone, 2 µM rosiglitazone, 500 µM IBMX, and 5 µg/ml insulin in growth media 2 days after reaching full confluence. 2 days later, media can be replaced by differentiation induction media with 2 µM rosiglitazone and 5 µg/ml insulin for 4 days with media change every 2 days. Biological cells can be kept in maintenance medium (growth medium with 5 µg/ml insulin) for 4 more days for full differentiation. One day before the experiments, biological cells can be washed with warm PBS and the media can be changed to growth media.

### 1.2 Administration of a lipolysis modulating agent

After differentiation and before and/or after Nile Red staining, i.e., before step (a) of the first round, lipolysis modulating agents can be administered to the adipocytes. The modulating agent can be a lipase inhibitor, as for example Atglistatin 10 µM and CAY10499 10 µM, or insulin 100 nM. In other embodiments, FGF-1 can be used as modulating agent.

In other embodiments, the lipolysis modulating agent can be administered after Nile Red staining, i.e., after step (a) of the first round.

### 1.3 Administration of a target

Insulin resistance in adipocytes can be induced by 3 days of dexamethasone administration at 100 nM before the experiment, i.e., before Nile Red staining, i.e. before step (a) of the first round. Since dexamethasone is known for its insulin-desensitizing effects, it can serve in this example as a placeholder for a target.

Insulin (re-) sensitization can be induced by co-treatment of dexamethasone-induced insulin resistant adipocytes for 24 h with 10 µM rosiglitazone during the last day of dexamethasone administration, i.e., before Nile Red staining, i.e. before step (a) of the first round. Since rosiglitazone is known for its insulin-sensitizing effects both in vitro and in vivo it can serve in this example as a placeholder for a target.

In other embodiments, the target can be administered after Nile Red staining, i.e., after step (a) of the first round.

### 1.4 Nile Red staining

On the day of the experiment, adipocytes can be washed with PBS and fasted in DMEM, 0.5% BSA, 10 mM Hepes, and antibiotic-antimycotic for 2.5 h. Afterwards, media can be changed to KRBH buffer (30 mM Hepes, 120 mM NaCl, 4 mM KH₂PO₄, 1 mM MgSO₄, 0.75 mM CaCl₂, and 10 mM NaHCOs), 5 mM glucose, 2 % BSA, and 200 ng/ml Nile Red, for 30 min. Then, adipocytes can be washed 4x with warm PBS and medium can be changed to KRBH buffer with 2 % FFA free BSA and 5 mM glucose.

### 1.5 Lipolysis determination

Isoproterenol can be administered to the adipocytes to induce lipolysis. In other preferred embodiments, PDE inhibitors can be used to induce lipolysis. Then, the plate can be transferred to an Incucyte S3 plate reader (Sartorius) inside a 37°C biological cell culture incubator. 9 pictures/well can be taken every hour for 8 h in each red channel and green channel. After having excited Nile Red for fluorescence at a first excitation wave length of 585 nm, a fluorescence intensity A in the red channel can be determined at a first emission wave length of 635 nm. After having excited Nile Red for fluorescence at a second excitation wave length of 460 nm, a fluorescence intensity B in the green channel can be determined at a second emission wave length of 524 nm. Furthermore, brightfield channel can be determined for cellular morphology.

In other preferred embodiments, the first excitation wave length is from 565 nm to 605 nm, preferably from 575 nm to 595 nm, most preferably 585 nm.

In other preferred embodiments, the first emission wave length is from 610 nm to 655 nm, preferably from 625 nm to 645 nm, most preferably 635 nm.

In other preferred embodiments, the second excitation wave length is from 440 nm to 490 nm, preferably from 450 nm to 470 nm, most preferably 460 nm.

In other preferred embodiments, the second emission wave length is from 500 nm to 550 nm, preferably from 515 nm to 535 nm, most preferably 524 nm.

Red to green ratio for each picture in each well can be calculated by dividing fluorescence intensity A by fluorescence intensity B. The ratio can be normalized to the respective ratio calculated in the first round (t = 0 h).

In other preferred embodiments, green to red ratio can be calculated by dividing fluorescence intensity B by fluorescence intensity A.

An integral of the ratio calculated in step (f) of each round over the time, i.e., the area under the curve (AUC), can be calculated by use of the computer-implemented software Prism using a sample with no administration of isoproterenol and no administration of manipulating substances as the baseline for normalization, such that the respective baseline integral is subtracted from each calculated integral.

### 1.6 Comparative example

As comparative example for a method to determine lipolysis, supernatant of the biological cells can be collected after 5 h of treatment and FFA levels can be determined in the medium using a commercially available, colorimetric, biochemical, enzyme-based kit (Wako-NEFAHR2, Fujifilm).

### 2. Results

### 2.1 Determination of lipolysis

Fig. 1 shows a diagram of the lipolytic status in 3T3L1 adipocytes after administration of 0 nM (dotted line), 10 nM (dashed line), 100 nM (dotted-dashed line), or 1000 nM (full line) isoproterenol and determination of the lipolysis according to the invention over a period of 8 h each hour, i.e., in 9 rounds, normalized to the ratio calculated in the first round. On the y-axis the ratio from fluorescence intensity A and fluorescence intensity B is shown. On the x-axis the time in hours is shown. Compared to the control sample, to which no isoproterenol was added, increased isoproterenol concentrations, up to 100 nM, saturated the maximal ratio at 2 h (Fig. 1). Higher (1000 nM) isoproterenol concentration did not change the peak but extended the duration of the enhanced lipolysis.

Fig. 2 shows a diagram of the data shown in Fig. 1 after calculation of an integral of the ratio calculated in step (f) of each round over the time. On the y-axis the integral of the ratio calculated in step (f) of each round over the time normalized to the sample with administration of 0 nM isoproterenol is shown. On the x-axis the concentration of isoproterenol in nM is shown. The integral over the time increases with increasing isoproterenol concentration.

Fig. 3 shows a diagram of concentration of FFA determined by a commercially available kit after 5 h in the samples of the experiment shown in Fig. 1 and Fig. 2 as a comparative example. On the y-axis the concentration of FFA is shown. On the x-axis the concentration of isoproterenol is shown. It is known that the concentration of FFA positively correlates with lipolysis. The concentration of FFA increases with increasing isoproterenol concentration.

Thus, the results obtained by application of the commercially available kit correlate with the results obtained by application of the method according to the invention. Thus, the ratio is indicative of the lipolytic status of the living biological cell, and the integral of the ratio calculated in step (f) of each round over the time is indicative of the lipolytic status of the living biological cell over the time from the first round to the at least second round.

### 2.2 Determination of lipolysis under modulation

Fig. 4 shows a diagram of the lipolytic status in 3T3L1 adipocytes after administration of isoproterenol (ISO), insulin (INS), and/or a lipase inhibitor (LIH) and determination of the lipolysis according to the invention over a period of 8 h each hour, i.e., in 9 rounds. On the y-axis the integral of the ratio calculated in step (f) of each round over the time normalized to the sample without administration of isoproterenol is shown. On the x-axis the treatment conditions are shown.

Fig. 5 shows a diagram of concentration of FFA determined by a commercially available kit after 5 h in the samples of the experiment shown in Fig. 4 as a comparative example. On the y-axis the concentration of FFA is shown. On the x-axis the treatment conditions are shown.

The results obtained by application of the commercially available kit (Fig. 5) correlate with the results obtained by application of the method according to the invention (Fig. 4). Thus, the method of the invention indeed allows intracellular determination of lipolysis in response to administration of a lipolysis modulating agent.

### 2.3 Detection of insulin resistance and insulin sensitization

Fig. 6 shows a diagram of the lipolytic status in 3T3L1 adipocytes after administration of isoproterenol (ISO), insulin (INS), dexamethasone (DEX) and/or rosiglitazone (ROS) and determination of the lipolysis according to the invention over a period of 8 h each hour, i.e., in 9 rounds. On the y-axis the integral of the ratio calculated in step (f) of each round over the time normalized to the sample without administration of isoproterenol is shown. On the x-axis the treatment conditions are shown.

Insulin robustly suppressed the lipolysis in control adipocytes, but had minimal effect in insulin resistant adipocytes. Administration of rosiglitazone partially relieved insulin resistant adipocytes.

Thus, the method according to the invention can be used to test a target for its ability to antagonize, inhibit, agonize, or induce the modulation of the lipolysis caused by a modulating agent.

The experiment can be performed in 48-well plates. In other preferred embodiments, other multi-well plates can be used, for example 96-well plates. Thus, the method allows to screen for potential insulin-sensitizers, and modulating agents in a high-throughput format.

### 3. Conclusion

The inventors provide a method that can be used to determine lipolysis in living biological cells in real-time, which method causes low costs, has short hands-on time allows kinetic determinations of lipolysis, re-usage of biological cells for other experiments, and quantification of lipid droplets, provides detailed information about biological cell morphology, and being high-throughput compatible.

## Claims

1. A method for determining the lipolytic status in a living biological cell, the method comprising
(a) contacting a biological cell and Nile Red,
(b) exciting said Nile Red for fluorescence at a first excitation wave length,
(c) determining a fluorescence intensity A emitted by said Nile Red at a first emission wave length,
(d) exciting said Nile Red for fluorescence at a second excitation wave length,
(e) determining a fluorescence intensity B emitted by said Nile Red at a second emission wave length, and
(f) calculating a ratio from the fluorescence intensity A and the fluorescence intensity B,
wherein the ratio calculated in step (f) is indicative of the lipolytic status of the living biological cell.

2. The method of claim 1, comprising the following further step after step (f):
(g) repeating steps (b) to (f) for an at least second round, wherein a period of time t has elapsed between the first round and the at least second round.

3. The method of claim 2, comprising the following further step after step (g):
(h) comparing the ratio calculated in step (f) in the first round with the ratio calculated in step (f) in the at least second round to determine the lipolytic status of the living biological cell over the time t.

4. The method of any one of the preceding claims, comprising the following further step before step (a):
(a') administering to the biological cell a substance inducing lipolysis, the substance being preferably selected from the group consisting of: β-adrenergic agonists, alpha-2-antagonists, PDE inhibitors, forskolin, and hormones, more preferably selected from the group consisting of: isoproterenol, PDE inhibitors, forskolin, catecholamines, glucagon, atrial natriuretic peptide (ANP), and brain natriuretic peptide (BNP).

5. The method of any one of the preceding claims, wherein the biological cell is an adipocyte.

6. The method of any one of the preceding claims, wherein the first excitation wave length is from 565 nm to 605 nm, preferably from 575 nm to 595 nm, most preferably 585 nm, and preferably the first emission wave length is from 610 nm to 655 nm, preferably from 625 nm to 645 nm, most preferably 635 nm.

7. The method of any one of the preceding claims, wherein the second excitation wave length is from 440 nm to 490 nm, preferably from 450 nm to 470 nm, most preferably 460 nm, and preferably the second emission wave length is from 500 nm to 550 nm, preferably from 515 nm to 535 nm, most preferably 524 nm.

8. The method of any one of the preceding claims, wherein the steps (b) to (f) are repeated for approximately 100 rounds, preferably at for approximately 10 rounds.

9. The method of any of claims 2 to 8, wherein each ratio calculated in step (f) of any round is normalized to a ratio calculated in step (f) of a reference round.

10. The method of claim 9, wherein the reference round is the first round.

11. The method of any one of the preceding claims, wherein at least step (f) and/or, if provided, at least step (h) is computer-implemented.

12. The method of any one of the preceding claims, wherein at least one of the steps (b) to (e) is conducted by means of a fluorescence microscope or a plate reader, preferably the steps (b) to (e).

13. The method of any one of the preceding claims, comprising:
(s) administering to the biological cell an agent to be tested for its ability to modulate the lipolytic status.

14. The method of any one of claims 1 to 12, comprising:
(t) administering to the biological cell a modulating agent modulating the lipolytic status, the modulating agent being preferably an anti-lipolytic hormone, more preferably selected from the group consisting of: insulin, fibroblast growth factor 1 (FGF-1), and insulin like growth factor 1 (IGF1).

15. The method of claim 14, comprising
(u) administering to the biological cell a target to be tested for its ability to antagonize, inhibit, agonize, or induce the modulation of the lipolytic status caused by the modulating agent, the target being preferably selected from the group consisting of: a potential insulin-sensitizer, a potential insulin-desensitizer, a potential lipase inhibitor, a potential lipolysis activator, and a potential lipolysis inhibitor.
